# FASCICULE DE BREVET EUROPEEN

(11) **EP 4 406 423 B1**
(45) Date de publication et mention de la délivrance du brevet: **13.08.2025**
(21) Numéro de dépôt: 24152538.5
(22) Date de dépôt: 18.01.2024
(51) Int. Cl.: A23N 1/00, B30B 9/02, C12G 1/00, G01N 27/416, B30B 9/22, B30B 15/26, G01N 33/14

(54) **PROCEDE ET INSTALLATION DE DETERMINATION EN AVAL D'UNE CHAMBRE DE PRESSURAGE D'UN PRESSOIR, DE LA NATURE DE JUS DE RAISIN PRODUITS ET ENSEMBLE COMPRENANT UN PRESSOIR ET UNE TELLE INSTALLATION**
VERFAHREN UND VORRICHTUNG ZUR BESTIMMUNG AM AUSLAUF EINER DRUCKKAMMER EINER PRESSE, DER ART VON PRODUZIERTEM TRAUBENSAFT, UND ANORDNUNG MIT EINER PRESSE UND EINER SOLCHEN VORRICHTUNG
METHOD AND INSTALLATION FOR DETERMINING DOWNSTREAM OF A PRESSING CHAMBER OF A PRESS, THE KIND OF GRAPE JUICE PRODUCED AND ASSEMBLY COMPRISING A PRESS AND SUCH AN INSTALLATION

(30) Priorité: 27.01.2023 FR 2300758; 27.01.2023 FR 2300759
(43) Date de publication de la demande: 31.07.2024
(73) Titulaire: Bucher Vaslin, 49290 Chalonnes sur Loire (FR)
(72) Inventeur: NOILET, Pascal, 49000 ANGERS (FR)
(74) Mandataire: Ipsilon

(56) Documents cités:
- EP-A1- 2 433 508
- WO-A1-2008/154409
- DE-A1- 19 924 475
- FR-A1- 3 051 101
- US-A- 4 253 390

## Description

La présente invention concerne un procédé et une installation de détermination, en aval d'une chambre de pressurage d'un pressoir, de la nature de jus produits au moins par pressurage de raisins dans ladite chambre de pressurage, ainsi qu'un ensemble comprenant un pressoir et une telle installation.

L'invention concerne en particulier un procédé de détermination, en aval d'une chambre de pressurage d'un pressoir, de la nature de jus produits au moins par pressurage de raisins dans ladite chambre de pressurage en vue de distinguer les jus issus de la zone de la pulpe des grains de raisin des jus issus de la zone de la pellicule des grains de raisin, ladite chambre de pressurage étant une chambre de volume variable apte à presser les raisins par variation de son volume, ledit procédé comprenant au moins une étape de mémorisation d'une valeur de pH de consigne et une étape de mesure en temps réel et en continu du pH des jus collectés en aval de la chambre de pressurage au moins jusqu'à obtention de ladite valeur de pH de consigne mémorisée

Dans une baie ou grain de raisin, on distingue la zone de la pulpe et la zone de la pellicule de la baie. On entend ici par pulpe la partie molle du grain de raisin à l'exclusion des pépins. Lors du pressurage, les jus sont obtenus sous l'effet d'une pression sur la baie entraînant l'éclatement de la baie. Ces jus comprennent des jus dits de la zone de la pulpe et des jus dits de la zone de la pellicule. Lorsque le référentiel est le rendement d'extraction (volume de jus/masse de la vendange), on considère que ce rendement est généralement voisin de 0,8, c'est-à-dire que pour 100 kg de vendange éraflée/foulée, on récupère 80 litres de jus. Sur ces 80 litres de jus, on considère que les 10 derniers litres de jus collectés sont des jus de la zone de la pellicule. Ces jus de la zone de la pellicule présentent une composition différente de celle des jus de la zone de pulpe et sont jugés moins qualitatifs. C'est pour cette raison qu'on essaye depuis de nombreuses années de séparer les jus extraits d'un pressoir comme l'illustre le brevet EP 2 433 508. La méthode repose sur la mesure en temps réel et en continu du pH en sortie de pressoir. Si cette méthode est appréciable, elle ne permet pas de séparer au plus près de la réalité les jus en fonction de la nature des jus. Les fabricants de matériels viticoles sont donc de manière permanente à la recherche de solutions permettant encore d'améliorer la finesse de la séparation des jus sans nuire à la simplicité de mise en œuvre.

Un but de l'invention est de proposer un procédé du type précité dont la mise en œuvre permet de séparer de manière précise les jus en fonction de leur nature.

Un autre but de l'invention est de proposer une installation notamment pour la mise en œuvre du procédé dont la conception permet une détermination fiable et précise de la nature des jus collectés sans augmenter de manière importante le coût de l'installation.

A cet effet, l'invention a pour objet un procédé de détermination, en aval d'une chambre de pressurage d'un pressoir, de la nature de jus produits au moins par pressurage de raisins dans ladite chambre de pressurage en vue de distinguer les jus issus de la zone de la pulpe des grains de raisin des jus issus de la zone de la pellicule des grains de raisin, ledit procédé comprenant au moins une étape de mémorisation d'une valeur de pH de consigne et une étape de mesure en temps réel et en continu du pH des jus collectés en aval de la chambre de pressurage au moins jusqu'à obtention de ladite valeur de pH de consigne mémorisée, caractérisé en ce que ledit procédé comprend, avant l'étape de mémorisation de ladite valeur de pH de consigne, une étape de mesure du pH et de la conductivité des jus collectés en aval de la chambre de pressurage pour enregistrer un couple de valeurs pH/conductivité et une étape de détermination de la valeur de pH de consigne à mémoriser en fonction du couple pH/conductivité mesuré.

La détermination de la valeur de pH de consigne en fonction d'une mesure du pH et de la conductivité à un instant déterminé par rapport au pressurage, avant le suivi en temps réel et en continu du pH des jus collectés, permet de définir avec plus de précision la valeur de pH de consigne correspondant au changement de nature des jus et au passage des jus issus de la zone de la pulpe au passage des jus issus de la zone de la pellicule.

Selon un mode de mise en œuvre du procédé de l'invention, ledit procédé comprend avant l'étape de mémorisation de la valeur de pH de consigne, une étape de mémorisation de données de pH et de conductivité de référence, et le procédé comprend, au cours de l'étape de détermination de la valeur de pH de consigne à mémoriser en fonction du couple pH/conductivité mesuré, une phase de comparaison des données de pH et de conductivité de référence avec le couple de valeurs pH/conductivité mesurées et une phase de détermination d'une augmentation du pH en fonction de la comparaison, la valeur de pH de consigne à mémoriser étant égale à la valeur de pH du couple de valeurs pH/conductivité mesurées additionnée de la valeur d'augmentation du pH résultant de la comparaison des données de pH et de conductivité de référence avec le couple de valeurs pH/conductivité mesurées. La mémorisation de données de pH et de conductivité de référence est réalisée par exemple sous forme d'abaques. Les valeurs de pH et de conductivité de référence peuvent être mémorisées en corrélation avec une valeur d'augmentation de pH. En variante ou en complément, la valeur d'augmentation de pH est déterminée par calcul en fonction du résultat de la comparaison des données de pH et de conductivité de référence avec le couple de valeurs de pH/conductivité mesurées. Le couple de valeurs pH/conductivité mesurées peut donc être comparé à des valeurs de pH et de conductivité de référence qui sont corrélées de manière empirique avec une valeur d'augmentation du pH pour déterminer, à partir du couple de valeurs pH/conductivité mesurées, la valeur d'augmentation du pH à ajouter au pH mesuré pour obtenir la valeur de pH de consigne.

Selon un mode de mise en œuvre du procédé de l'invention, il comprend une étape d'émission d'un signal d'alerte sonore et/ou lumineux lorsque la valeur de pH de consigne est atteinte. Le viticulteur est ainsi informé que la nature des jus collectés vient de changer et il peut, si nécessaire, agir en conséquence en particulier en interrompant la collecte des jus ou en modifiant la trajectoire du flux de jus collectés ou le réservoir de collecte.

Selon un mode de mise en œuvre du procédé de l'invention, les jus produits au moins par pressurage de raisins dans ladite chambre de pressurage étant collectés en aval de la chambre de pressurage en suivant une première ou une seconde voie de circulation, le procédé comprend une étape de changement de la voie de circulation lorsque la valeur de consigne de pH est atteinte.

Selon un mode de mise en œuvre du procédé de l'invention, le procédé comprend une étape d'interruption de l'étape de collecte des jus lorsque la valeur de pH de consigne est atteinte.

Selon un mode de mise en œuvre du procédé de l'invention, ladite chambre de pressurage étant une chambre de volume variable apte à presser les raisins par variation de son volume et le pressurage comprenant une succession de paliers de pression, avec chaque palier correspondant à l'application d'une pression résultant d'une réduction de volume de la chambre de pressurage pendant une période de temps, ledit procédé comprend au moins une étape de détermination de l'avancement du pressurage et l'étape de mesure du pH et de la conductivité des jus collectés en aval de la chambre de pressurage pour enregistrer un couple de valeurs pH /conductivité est réalisée en fonction de l'avancement dudit pressurage. Idéalement la mesure du pH et de la conductivité pour former le couple de valeurs pH/conductivité s'opère pendant le premier palier de pression pour une vendange éraflée-foulée ou plus tardivement au cours du pressurage dans le cas d'une vendange entière.

Selon un mode de mise en œuvre du procédé de l'invention, le procédé comprend, au cours de l'étape de détermination de l'avancement du pressurage, au moins une phase de mesure directe ou indirecte de l'état de la déformation de la chambre de pressurage.

Selon un mode de mise en œuvre du procédé de l'invention, la chambre de pressurage de volume variable étant une chambre délimitée au moins partiellement par une membrane déformable en fonction de la pression intérieure d'une chambre de commande séparée de la chambre de pressurage au moins par ladite membrane, le procédé comprend, au cours de l'étape de détermination de l'avancement du pressurage, au moins une phase de mesure de la pression à l'intérieur de ladite chambre de commande. Le simple suivi de la pression permet de déterminer le stade d'avancement du pressurage pour détecter l'instant de mesure du couple de valeurs pH/conductivité.

Selon un mode de mise en œuvre du procédé de l'invention, le procédé comprend au moins une étape d'addition dans les jus collectés en aval de la chambre de pressurage d'au moins un agent dit de collage à fonction d'agent de floculation ou de coagulation d'au moins une partie des composés desdits jus de raisin. Il est ainsi possible du fait de la possibilité de déterminer la nature des jus collectés de procéder directement, lors de la collecte, à un traitement d'une partie des jus collectés en particulier les jus issus de la zone de la pellicule pour pouvoir éventuellement les remélanger ensuite avec les jus issus de la zone de la pulpe.

L'invention a encore pour objet, une installation de détermination, en aval d'une chambre de pressurage d'un pressoir, de la nature de jus de raisin produits au moins par pressurage de raisin dans ladite chambre de pressurage en vue de distinguer les jus issus de la zone de la pulpe des grains de raisin des jus issus de la zone de la pellicule des grains de raisin, ladite installation comprenant au moins, un système de collecte de jus disposé en aval de la chambre de pressurage et apte à être en communication fluidique avec la chambre de pressurage pour collecter les jus produits par pressurage du raisin dans la chambre de pressurage, au moins un capteur de mesure de pH disposé au niveau du système de collecte, au moins une mémoire de stockage de données et une unité de pilotage configurée pour commander le stockage d'une valeur de pH de consigne dans la ou l'une des mémoires et la mesure en temps réel et en continu du pH au niveau du système de collecte au moins jusqu'à obtention de ladite valeur de pH de consigne mémorisée, caractérisée en ce que l'installation comprend, au niveau du système de collecte, un capteur de mesure de la conductivité et en ce que l'unité de pilotage est configurée pour, préalablement à la commande du stockage d'une valeur de pH de consigne dans la ou l'une des mémoires, commander la mesure, au niveau du système de collecte, du pH et de la conductivité pour enregistrer un couple de valeurs pH /conductivité et pour déterminer la valeur de pH de consigne à mémoriser en fonction du couple de valeurs pH/conductivité mesurées. L'installation permet donc notamment la mise en œuvre du procédé tel que décrit ci-dessus.

Selon un mode de réalisation de l'invention, la ou au moins l'une des mémoires est une mémoire de stockage de données de pH et de conductivité de référence, et l'unité de pilotage est configurée pour comparer les données de pH et de conductivité de référence avec le couple de valeurs pH/conductivité mesurées et pour déterminer une augmentation du pH en fonction de la comparaison, la valeur de pH de consigne à mémoriser étant égale à la valeur de pH du couple de valeurs pH/conductivité mesurées additionnée de la valeur d'augmentation du pH résultant de la comparaison des données de pH et de conductivité de référence avec le couple de valeurs de pH/conductivité mesurées.

Les données de pH et de conductivité peuvent être mémorisée sous forme d'abaques. Les données de pH et de conductivité de référence peuvent être mémorisées en corrélation avec une valeur d'augmentation de pH.

En variante ou en complément, l'augmentation du pH est déterminée par calcul en fonction de la comparaison entre les données de pH et de conductivité de référence avec le couple de valeurs pH/conductivité mesurées.

Selon un mode de réalisation de l'invention, l'installation comprend au moins un dispositif d'émission d'un signal d'alerte sonore et/ou lumineux et l'unité de pilotage est configurée pour commander l'émission d'un signal d'alerte sonore et/ou lumineux par le dispositif d'émission d'un signal d'alerte sonore et/ou lumineux lorsque la valeur de pH de consigne est atteinte.

Selon un mode de réalisation de l'invention, le système de collecte de jus comprenant un réservoir raccordable en entrée à la chambre de pressurage, ledit réservoir est équipé en sortie d'une première voie de circulation et d'une seconde voie de circulation sélectivement obturables, et l'unité de pilotage est configurée pour commander l'obturation de l'une des voies de circulation et l'ouverture de l'autre voie de circulation lorsque la valeur de pH de consigne est atteinte.

Selon un mode de réalisation de l'invention, le système de collecte de jus comprenant un réservoir raccordable en entrée à la chambre de pressurage, ladite entrée du réservoir est équipée d'un organe d'obturation et l'unité de pilotage est configurée pour commander la fermeture de l'organe d'obturation de ladite entrée lorsque la valeur de pH de consigne est atteinte. Cette fermeture de l'organe d'obturation peut être accompagnée d'une mise en pause du pressoir nécessitant une réactivation manuelle du pressoir par un opérateur.

Selon un mode de réalisation de l'invention, le pressurage comprenant une succession de paliers de pression, avec chaque palier correspondant à l'application d'une pression pendant une période de temps, ladite installation comprend au moins un système de détermination de l'avancement du pressurage et l'unité de pilotage est configurée pour mesurer le pH et la conductivité des jus collectés en aval de la chambre de pressurage pour enregistrer un couple de valeurs pH/conductivité en fonction de l'avancement dudit pressurage.

Selon un mode de réalisation de l'invention, la chambre de pressurage étant une chambre de volume variable apte à presser les raisins par variation de son volume, le système de détermination de l'avancement du pressurage comprend au moins un organe de mesure directe ou indirecte de l'état de la déformation de la chambre de pressurage.

Selon un mode de réalisation de l'invention, la chambre de pressurage étant une chambre délimitée au moins partiellement par une membrane déformable en fonction de la pression intérieure d'une chambre de commande séparée de la chambre de pressurage au moins par ladite membrane, le ou au moins l'un des organes de mesure directe ou indirecte de l'état de la déformation de la chambre du système de détermination de l'avancement du pressurage est un organe de mesure de la pression de préférence à l'intérieur de ladite chambre de commande.

Selon un mode de réalisation de l'invention, le système de collecte des jus qui comprend un réservoir dit principal raccordable en entrée à la chambre de pressurage comprend au moins un réservoir dit auxiliaire pour le stockage d'agents dits de collage à fonction d'agent de floculation ou de coagulation d'au moins une partie des composés desdits jus de raisin, et un système de transfert du contenu du réservoir auxiliaire vers le réservoir principal.

Selon un mode de réalisation de l'invention, l'installation comprend un dispositif de mesure de la quantité de jus collectés dans le réservoir principal, tel qu'un débitmètre, et l'unité de pilotage est configurée pour commander le système de transfert en fonction au moins des données fournies par le dispositif de mesure.

L'invention a encore pour objet un ensemble comprenant un pressoir comprenant une chambre de pressurage et une installation de détermination , en aval de ladite chambre de pressurage du pressoir, de la nature de jus de raisin produits au moins par pressurage de raisin dans ladite chambre de pressurage en vue de distinguer les jus issus de la zone de la pulpe des grains de raisin des jus issus de la zone de la pellicule des grains de raisin, ladite installation comprenant au moins, un système de collecte de jus disposé en aval de la chambre de pressurage et apte à être en communication fluidique avec la chambre de pressurage pour collecter au moins les jus produits par pressurage du raisin dans la chambre de pressurage, au moins un capteur de mesure de pH disposé au niveau du système de collecte, au moins une mémoire de stockage de données et une unité de pilotage configurée pour commander le stockage d'une valeur de pH de consigne dans la ou l'une des mémoires et la mesure en temps réel et en continu du pH au niveau du système de collecte au moins jusqu'à obtention de ladite valeur de pH de consigne mémorisée, caractérisé en ce que ladite installation est conforme à celle décrite ci-dessus.

### Brève description des dessins

L'invention sera bien comprise à la lecture de la description suivante d'exemples de réalisation, en référence aux dessins annexés dans lesquels :
[Fig. 1] représente une vue schématique d'une installation conforme à l'invention associée à un pressoir pour former un ensemble conforme à l'invention ;
[Fig. 2] représente une courbe pression/temps d'un pressurage et illustrant l'instant de mesure du couple pH/conductivité.

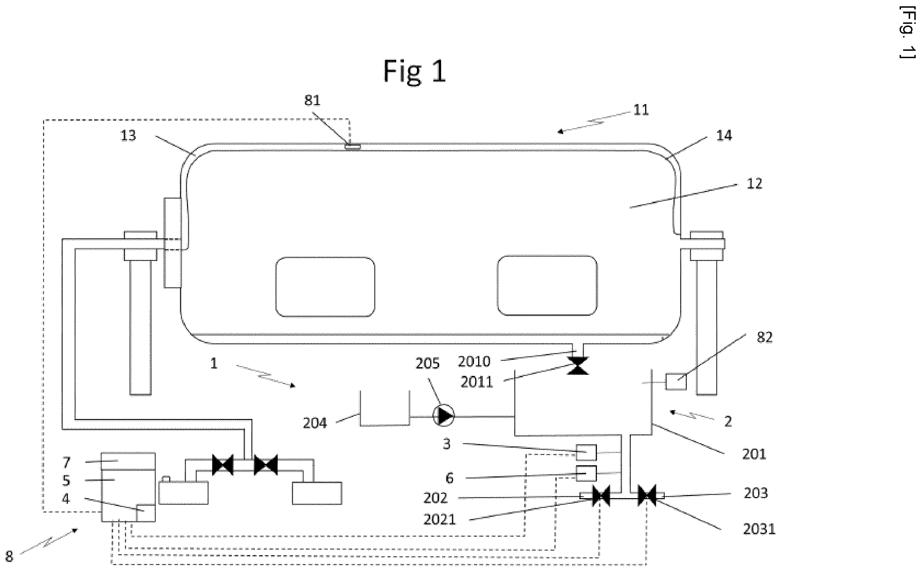

Comme mentionné ci-dessus, l'invention concerne un procédé de détermination, en aval d'une chambre de pressurage d'un pressoir 11, de la nature de jus produits au moins par pressurage dans la chambre 12 de pressurage, en vue de distinguer les jus issus de la zone de la pulpe de grains de raisin de ceux issus de la zone de la pellicule des grains de raisins qui sont extraits de la chambre de pressurage en fin de pressurage, ainsi qu'une installation 1 du type de celle représentée à la figure 1 pour la mise en œuvre du procédé, cette installation 1 formant, à l'état en communication fluidique avec le pressoir 11, un ensemble également objet de l'invention.

Le pressoir 11, avec lequel l'installation 1 est destinée à être en communication fluidique, peut être un pressoir 11 de type pneumatique à membrane 14, comme dans l'exemple représenté ou un pressoir à vis ou à plateau ou autre.

Indifféremment du type de pressoir 11, ce dernier présente une chambre 12 de pressurage, c'est-à-dire une chambre de volume variable à l'intérieur de laquelle la vendange entière ou éraflée et foulée est déversée.

Le pressurage, c'est-à-dire le pressage de la vendange en vue d'extraire les jus, s'opère par variation du volume de la chambre 12 de pressurage dans le sens d'une réduction du volume, de manière en soi connue.

Dans l'exemple représenté à la figure 1, la chambre 12 de pressurage est une chambre délimitée partiellement par une membrane 14 déformable. En effet, le pressoir comprend une cuve logeant une membrane 14 qui sépare la cuve en la chambre 12 de pressurage et une chambre de commande 13.

Cette chambre de commande 13 est séparée de la chambre 12 de pressurage par la membrane 14. Cette chambre de commande 13 peut être mise sous pression par raccordement à une source d'air comprimé. Cette mise sous pression de la chambre 13 de commande entraîne une déformation de la membrane 14 dans le sens d'une réduction de volume de la chambre 12 de pressurage. Cette chambre 13 de commande peut également être mise en dépression pour un retour de la membrane à son état non déformé dans lequel elle est plaquée contre une paroi de la cuve, comme illustré à la figure 1.

La chambre 12 de pressurage est équipée d'une sortie des jus en aval de laquelle vient se positionner un système 2 de collecte de jus de l'installation 1. Ce système 2 de collecte de jus est apte à être en communication fluidique avec la chambre 12 de pressurage pour collecter les jus produits par pressurage du raisin de la chambre 12 de pressurage. La communication fluidique peut s'opérer avec ou sans dispositif de raccordement entre la chambre 12 de pressurage et le système 2 de collecte. En l'absence de dispositif de raccordement, il suffit de disposer le système 2 de collecte, en particulier le réservoir 201 du système 2 de collecte qui sera décrit ci-après, sous la sortie des jus de la chambre 12 de pressurage. Les jus chutent ainsi directement dans le système 2 de collecte. Ce système 2 de collecte de jus comprend donc dans au moins un réservoir 201 dont l'entrée s'étend idéalement au-dessous et à la verticale de la sortie des jus de la chambre 12 de pressurage, comme illustré à la figure 1, pour une collecte gravitaire des jus issus du pressurage. Ce réservoir 201 est appelé réservoir principal lorsque l'installation 1 comprend plusieurs réservoirs.

L'entrée du réservoir 201 est représentée en 2010. Cette entrée 2010 du réservoir 201 peut être équipée d'un organe 2011 d'obturation, tel qu'une vanne. Dans l'exemple représenté, le réservoir 201 est équipé en sortie d'une première voie 202 de circulation et d'une seconde voie 203 de circulation. Chaque voie de circulation est équipée d'un organe d'obturation, tel qu'une vanne.

Ainsi, la première voie 202 de circulation est équipée d'un organe 2021 d'obturation, tandis que la seconde voie 203 de circulation est équipée d'un organe 2031 d'obturation. Les organes d'obturation sont sélectivement activables pour permettre l'obturation de l'une ou l'autre des voies.

En pratique, au cours du pressurage, l'une des voies de circulation est ouverte lors de la collecte d'une première nature de jus, à savoir par exemple, la collecte des jus issus de la zone de la pulpe des grains de raisins, tandis que l'autre voie de circulation est ouverte lors de la collecte des jus issus de la zone de la pellicule des grains de raisin.

Il est également possible d'obturer les deux voies lorsqu'il est souhaité opérer un traitement des jus directement dans le réservoir 201, dit principal, disposé en amont desdites voies. A cet effet, le système 2 de collecte de jus peut comprendre en sus du réservoir 201 principal, en communication fluidique en entrée avec la chambre 12 de pressurage, un réservoir 204, dit auxiliaire, pour le stockage d'agents dits de collage à fonction d'agents de floculation ou de coagulation d'au moins une partie des composés des jus de raisin, et un système de transfert 205 du contenu du réservoir auxiliaire 204 vers le réservoir principal 201. Ce système de transfert 205 peut comprendre une pompe doseuse comme illustré à la figure 1.

L'installation 1 comprend encore au moins un capteur de mesure de pH représenté en 3 et disposé au niveau du système 2 de collecte. Idéalement, ce capteur de mesure de pH 3 formé, par exemple par une sonde de mesure, est disposé au niveau du réservoir 201 principal et mesure à son niveau le pH des jus collectés.

L'installation 1 comprend encore au moins une mémoire 4 de stockage de données et une unité 5 de pilotage. Ladite unité 5 de pilotage se présente sous la forme d'un système électronique et informatique qui comprend par exemple un microprocesseur et une mémoire de travail. Selon un aspect particulier, l'unité de pilotage peut se présenter sous la forme d'un automate programmable. Autrement dit, les fonctions et étapes décrites peuvent être mise en œuvre sous forme de programme informatique ou via des composants matériels (p. ex. des réseaux de portes programmables). En particulier, les fonctions et étapes opérées par l'unité de pilotage ou ses modules peuvent être réalisées par des jeux d'instructions ou modules informatiques implémentés dans un processeur ou contrôleur ou être réalisées par des composants électroniques dédiés ou des composants de type circuit logique programmable (ou FPGA qui est l'acronyme de l'anglais field-programmable gate array, ce qui correspond littéralement à réseau de portes programmable in-situ) ou de type circuit intégré propre à une application (ou ASIC qui est l'acronyme de l'anglais application-specific integrated circuit, ce qui correspond littéralement à circuit intégré spécifique à une application). Il est aussi possible de combiner des parties informatiques et des parties électroniques. Lorsqu'il est précisé que l'unité ou des moyens ou modules de ladite unité sont configurés pour réaliser une opération donnée, cela signifie que l'unité comprend des instructions informatiques et les moyens d'exécution correspondants qui permettent de réaliser ladite opération et/ou que l'unité comprend des composants électroniques correspondants.

L'installation comprend encore au niveau du système 2 de collecte, un capteur de mesure de la conductivité 6. Ce capteur de mesure de la conductivité 6 peut, de manière similaire au capteur de mesure de pH 3, être disposé au niveau du réservoir 201 principal pour mesurer à ce niveau la conductivité des jus collectés.

L'unité 5 de pilotage est configurée pour acquérir des données, en particulier des capteurs décrits ci-dessus, pour déterminer une valeur de pH de consigne, mémoriser cette valeur de pH de consigne et mesurer en temps réel et en continu le pH au niveau du système 2 de collecte jusqu'à obtention de la valeur de pH de consigne mémorisée. Cette valeur de pH de consigne est considérée comme correspondant à un changement de nature des jus au niveau des jus collectés.

Pour permettre la détermination d'une valeur de pH de consigne, l'unité 5 de pilotage est configurée pour enregistrer un couple de valeurs pH/conductivité, c'est-à-dire un couple de valeurs comprenant une valeur de pH et une valeur de conductivité, et pour déterminer la valeur de pH de consigne à mémoriser, en fonction du couple de valeurs pH/conductivité mesurées.

L'enregistrement du couple de valeurs pH/conductivité s'opère à un instant T1 en fonction de l'avancement du pressurage. Cet instant T1 peut être déterminé par l'opérateur qui peut visuellement déterminer que la phase d'égouttage est terminée et que le pressurage peut commencer. L'opérateur peut alors décider manuellement de lancer l'opération de pressurage et décider de l'instant, au cours de ce pressurage, auquel l'installation 1 doit enregistrer le couple de valeurs pH/conductivité. Dans ce cas, l'opérateur commande manuellement l'enregistrement d'un couple de valeurs pH/conductivité.

En variante, le déclenchement de cet enregistrement d'un couple de valeurs pH/conductivité peut être réalisé de manière automatique. A cet effet, l'installation 1 peut comprendre un système 8 de détermination de l'avancement de pressurage et l'unité 5 de pilotage est configurée pour mesurer le pH et la conductivité des jus collectés en aval de la chambre 12 de pressurage, pour enregistrer un couple de valeurs pH/conductivité en fonction de l'avancement dudit pressurage.

La commande de cet enregistrement peut par exemple s'opérer sur la base d'une mesure de débit des jus collectés. Le système 8 de détermination de l'avancement du pressurage peut donc comprendre un dispositif de mesure de la quantité de jus collecté. A cet effet, l'installation 1 peut comporter, en entrée du réservoir principal, un débitmètre représenté en 82 à la figure 1.

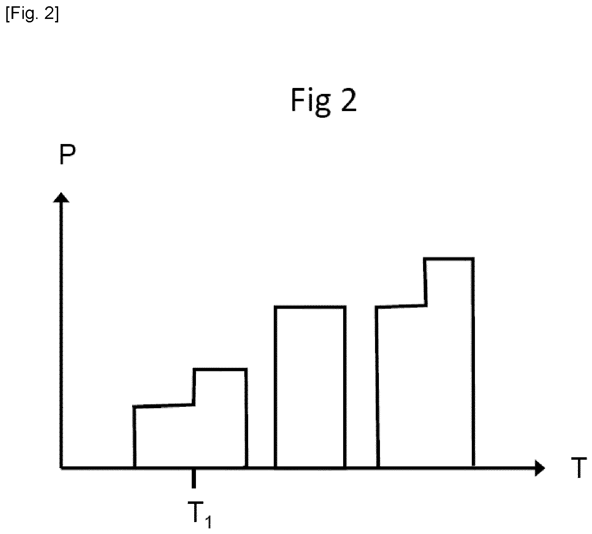

Généralement, le pressurage comprend une succession de paliers de pression avec chaque palier correspondant à l'application d'une pression P pendant une période de temps T, comme dans l'exemple représenté à la figure 2, qui illustre l'évolution de la pression P dans la chambre de commande en fonction du temps T. Les paliers sont mémorisés. L'installation 1 peut donc comprendre une mémoire dans laquelle sont stockés ces paliers de pression, c'est-à-dire les valeurs de pression et de temps.

Le système 8 de détermination de l'avancement de pressurage peut comprendre, quant à lui, au moins un organe 81 de mesure directe ou indirecte de l'état de la déformation de la chambre12 de pressurage.

Dans le cas d'un pressoir tel que décrit ci-dessus, cet organe 81 de mesure directe ou indirecte de l'état de la déformation de la chambre de pressurage du système 8 de détermination de l'avancement du pressurage, est un organe de mesure de la pression disposé de préférence à l'intérieur de la chambre de commande 13.

La mesure de l'état de la déformation de la chambre 12 de pressurage est donc, dans ce cas, une mesure indirecte.

L'installation 1 peut en effet ainsi comprendre un simple capteur de pression positionnable à l'intérieur de la chambre 13 de commande du pressoir. L'unité 5 de pilotage est configurée pour comparer la pression mesurée avec une pression de pressurage mémorisée et lorsque la pression de pressurage mémorisée est atteinte, les valeurs de pH et de conductivité mesurées à cet instant, sont mémorisées pour former le couple de valeurs pH/conductivité mesurées.

En pratique, l'instant T1, tel qu'illustré à la figure 2, correspond à l'instant d'enregistrement du couple de valeurs pH/conductivité. Il doit être rappelé qu'au cours du pressurage, après l'application d'un ou plusieurs paliers de pression, il est procédé à une mise sous-vide de la chambre de commande, puis éventuellement à un rebêchage, c'est-à-dire à une rotation de la cuve du pressoir avant de procéder à nouveau à l'application d'un ou plusieurs paliers de pression.

L'instant T1 d'enregistrement du couple de valeurs pH/conductivité mesurées correspond, dans le cas d'une vendange éraflée foulée à la première mise en pression de la chambre de commande, c'est-à-dire au premier palier de pression. Dans le cas d'une vendange entière, cet instant T1 correspond à la fin du premier cycle de pressurage, un cycle étant une succession de paliers de pression avant un rebêchage. On peut avoir un cycle suivant
5 min à 5 000 Pa
5 min à 10 000 Pa
5 min à 15 000 Pa
puis tirage au vide et rebêchage. Les trois paliers de pression correspondent à un cycle de pressurage. Dans un pressurage, il peut y avoir plusieurs cycles.

La ou l'une des mémoires 4 de l'installation est une mémoire de stockage de données de pH et de conductivité de référence, par exemple sous forme d'abaque. Ainsi, cette mémoire comprend, pour une valeur ou une plage de valeurs de conductivité exprimée par exemple en MilliSiemens par centimètre (mS/cm), et une valeur ou une plage de valeurs de pH, une valeur de Δ d'augmentation du pH.

Ainsi, par exemple, la mémoire peut comprendre les données suivantes :

**[Table 1]**

| Conductivité (mS/cm) | pH | Δ d'augmentation de pH |
|---|---|---|
| < 3,00 | < 3,5 | + 0,1 |
| > 3,01 | > 3,51 | + 0,05 |

L'unité 5 de pilotage est configurée pour comparer les données de pH et de conductivité de référence avec le couple de valeurs pH/conductivité mesurées et pour déterminer une augmentation du pH en fonction de la comparaison.

La valeur de pH de consigne à mémoriser est égale à la valeur de pH du couple de valeurs pH/conductivité mesurées, additionnée de la valeur d'augmentation du pH résultant de la comparaison des données de pH et de conductivité de référence avec le couple de valeurs de pH/conductivité mesurées.

Ainsi, par exemple, on suppose que le couple de valeurs pH/conductivité mesuré à T1 est égal à 3,2 pour le pH et à 2,90 pour la conductivité. Comme la conductivité mesurée est < 3, et le pH est < 3, alors la valeur de delta (Δ) d'augmentation du pH est égale à 0,1, de sorte que la valeur de consigne est égale à la valeur de pH mesurée plus le delta d'augmentation de pH soit 3,2+0,1=3,3. Cette valeur de pH de consigne égale à 3,3 est donc mémorisée, et l'unité 5 de pilotage acquiert, à compter de cette mémorisation en temps réel et en continu, la valeur de pH mesurée par le capteur de mesure de pH 3, au moins jusqu'à obtention de la valeur de pH de consigne.

Lorsque la valeur de pH de consigne est atteinte, il peut être envisagé plusieurs scénarios complémentaires ou cumulatifs. Ainsi, l'étape de collecte des jus peut être interrompue lorsque la valeur de pH de consigne est atteinte et le pressoir peut être mis en pause jusqu'à une réactivation manuelle par l'opérateur. En variante ou en complément, l'installation 1 peut comprendre un dispositif 7 d'émission d'un signal d'alerte sonore et/ou lumineux, tel qu'une alarme ou un terme témoin visuel, disposé à proximité ou au niveau du système 2 de collecte.

L'unité 5 de pilotage est configurée pour commander l'émission d'un signal d'alerte sonore et/ou lumineux par le dispositif 7 d'émission d'un signal d'alerte sonore et/ou lumineux, lorsque la valeur de pH de consigne est atteinte.

L'opérateur peut ainsi, lors de l'émission de ce signal, décider de stopper la collecte des jus ou de modifier les conditions de collecte. Ces opérations peuvent également s'opérer de manière automatique. Ainsi, l'unité 5 de pilotage peut être configurée pour commander la fermeture de l'organe 2011 d'obturation de l'entrée 2010 du réservoir 201 principal lorsque la valeur de pH de consigne est atteinte. Cette fermeture de l'organe 2011 d'obturation peut s'opérer avec ou sans émission d'un signal d'alerte sonore et/ou lumineux et avec de préférence une mise en pause du pressoir pouvant dans certains cas nécessiter une réactivation manuelle du pressoir.

L'unité 5 de pilotage peut encore commander l'obturation de la première voie 202 de circulation et l'ouverture de la seconde voie 203 de circulation, ou inversement, lorsque la valeur de pH de consigne est atteinte. De la même manière, cette opération peut s'opérer en parallèle de l'émission d'un signal d'alerte sonore et/ou lumineux ou non.

Une fois que cette valeur de pH de consigne est atteinte, il peut également être décidé d'interrompre la collecte et d'ajouter dans le réservoir 201 principal des agents de collage choisis de préférence dans le groupe formé par les produits de collage de type minéral, tels qu'une bentonite, un gel de silice ou de type organique, tels que de la gélatine, une caséine, une protéine de pois et stockés dans le réservoir 204 auxiliaire.

L'unité 5 de pilotage peut ainsi être configurée pour commander le système de transfert 205 pour une alimentation en automatique du réservoir 201 principal à partir du réservoir 204 auxiliaire. A cet effet, l'installation peut comprendre un dispositif de mesure de la quantité de jus collectés dans le réservoir 201 principal. Ce dispositif de mesure peut être formé par le débitmètre 82 décrit ci-dessus et disposé en entrée du réservoir 201 principal. L'unité 5 de pilotage est configurée pour commander le système de transfert 205 et en particulier la pompe doseuse du système de transfert 205 en fonction au moins des données fournies par le dispositif de mesure au regard de la consigne fournie par l'opérateur.

Grâce aux caractéristiques de l'installation 1, telle que décrite ci-dessus, et au procédé mis en œuvre, il en résulte une possibilité de déterminer de manière fiable et rapide la nature des jus collectés pour déterminer avec précision le moment lors de la collecte où les jus ne doivent plus être considérés comme des jus issus de la zone de la pulpe, mais comme des jus issus de la zone de la pellicule des grains de raisins.

Selon le niveau d'automatisation requis, l'installation 1 de détermination de la nature des jus, telle que décrite ci-dessus, pourra être entièrement autonome ou réalisée sous forme d'un ensemble avec le pressoir.

## Revendications

1. Procédé de détermination, en aval d'une chambre (12) de pressurage d'un pressoir (11), de la nature de jus produits au moins par pressurage de raisins dans ladite chambre (12) de pressurage en vue de distinguer les jus issus de la zone de la pulpe des grains de raisin des jus issus de la zone de la pellicule des grains de raisin, ledit procédé comprenant au moins une étape de mémorisation d'une valeur de pH de consigne et une étape de mesure en temps réel et en continu du pH des jus collectés en aval de la chambre (12) de pressurage au moins jusqu'à obtention de ladite valeur de pH de consigne mémorisée, **caractérisé en ce que** ledit procédé comprend, avant l'étape de mémorisation de ladite valeur de pH de consigne, une étape de mesure du pH et de la conductivité des jus collectés en aval de la chambre (12) de pressurage pour enregistrer un couple de valeurs pH /conductivité et une étape de détermination de la valeur de pH de consigne à mémoriser en fonction du couple pH/conductivité mesuré.

2. Procédé selon la revendication 1, **caractérisé en ce qu'**il comprend, avant l'étape de mémorisation de la valeur de pH de consigne, une étape de mémorisation de données de pH et de conductivité de référence, et **en ce que** le procédé comprend, au cours de l'étape de détermination de la valeur de pH de consigne à mémoriser en fonction du couple pH/conductivité mesuré, une phase de comparaison des données de pH et de conductivité de référence avec le couple de valeurs pH/conductivité mesurées et une phase de détermination d'une augmentation du pH en fonction de la comparaison, la valeur de pH de consigne à mémoriser étant égale à la valeur de pH du couple de valeurs pH/conductivité mesurées additionnée de la valeur d'augmentation du pH résultant de la comparaison des données de pH et de conductivité de référence avec le couple de valeurs pH/conductivité mesurées.

3. Procédé selon l'une des revendications 1 ou 2, **caractérisé en ce qu'**il comprend une étape d'émission d'un signal d'alerte sonore et/ou lumineux lorsque la valeur de pH de consigne est atteinte.

4. Procédé selon l'une des revendications 1 à 3, **caractérisé en ce que** les jus produits au moins par pressurage de raisins dans ladite chambre (12) de pressurage étant collectés en aval de la chambre (12) de pressurage en suivant une première (202) ou une seconde voie (203) de circulation, le procédé comprend une étape de changement de la voie de circulation lorsque la valeur de consigne de pH est atteinte.

5. Procédé selon l'une des revendications 1 à 4, **caractérisé en ce que** le procédé comprend une étape d'interruption de l'étape de collecte des jus lorsque la valeur de pH de consigne est atteinte.

6. Procédé selon l'une des revendications 1 à 5, **caractérisé en ce que** ladite chambre (12) de pressurage étant une chambre de volume variable apte à presser les raisins par variation de son volume et le pressurage comprenant une succession de paliers de pression, avec chaque palier correspondant à l'application d'une pression résultant d'une réduction de volume de la chambre de pressurage pendant une période de temps, ledit procédé comprend au moins une étape de détermination de l'avancement du pressurage et **en ce que** l'étape de mesure du pH et de la conductivité des jus collectés en aval de la chambre de pressurage pour enregistrer un couple de valeurs pH /conductivité est réalisée en fonction de l'avancement dudit pressurage.

7. Procédé selon la revendication 6, **caractérisé en ce que** le procédé comprend, au cours de l'étape de détermination de l'avancement du pressurage, au moins une phase de mesure directe ou indirecte de l'état de la déformation de la chambre (12) de pressurage.

8. Procédé selon l'une des revendications 6 ou 7, **caractérisé en ce que** la chambre (12) de pressurage de volume variable étant une chambre délimitée au moins partiellement par une membrane (14) déformable en fonction de la pression intérieure d'une chambre (13) de commande séparée de la chambre (12) de pressurage au moins par ladite membrane (14), le procédé comprend, au cours de l'étape de détermination de l'avancement du pressurage, au moins une phase de mesure de la pression à l'intérieur de ladite chambre (13) de commande.

9. Procédé selon l'une des revendications 1 à 8, **caractérisé en ce que** le procédé comprend au moins une étape d'addition dans les jus collectés en aval de la chambre (12) de pressurage d'au moins un agent dit de collage à fonction d'agent de floculation ou de coagulation d'au moins une partie des composés desdits jus de raisin.

10. Installation (1) de détermination, en aval d'une chambre (12) de pressurage d'un pressoir (11), de la nature de jus de raisin produits au moins par pressurage de raisin dans ladite chambre (12) de pressurage en vue de distinguer les jus issus de la zone de la pulpe des grains de raisin des jus issus de la zone de la pellicule des grains de raisin, ladite installation (1) comprenant au moins, un système (2) de collecte de jus disposé en aval de la chambre (12) de pressurage et apte à être en communication fluidique avec la chambre (12) de pressurage pour collecter les jus produits par pressurage du raisin dans la chambre (12) de pressurage, au moins un capteur de mesure de pH (3) disposé au niveau du système (2) de collecte, au moins une mémoire (4) de stockage de données et une unité (5) de pilotage configurée pour commander le stockage d'une valeur de pH de consigne dans la ou l'une des mémoires (4) et la mesure en temps réel et en continu du pH au niveau du système (2) de collecte au moins jusqu'à obtention de ladite valeur de pH de consigne mémorisée, **caractérisée en ce que** l'installation (1) comprend, au niveau du système (2) de collecte, un capteur de mesure de la conductivité (6) et **en ce que** l'unité (5) de pilotage est configurée pour, préalablement à la commande du stockage d'une valeur de pH de consigne dans la ou l'une des mémoires (4), commander la mesure, au niveau du système (2) de collecte, du pH et de la conductivité pour enregistrer un couple de valeurs pH /conductivité et pour déterminer la valeur de pH de consigne à mémoriser en fonction du couple de valeurs pH/conductivité mesurées.

11. Installation (1) selon la revendication 10, **caractérisée en ce que** la ou au moins l'une des mémoires est une mémoire de stockage de données de pH et de conductivité de référence, et **en ce que** l'unité (5) de pilotage est configurée pour comparer les données de pH et de conductivité de référence avec le couple de valeurs pH/conductivité mesurées et pour déterminer une augmentation du pH en fonction de la comparaison, la valeur de pH de consigne à mémoriser étant égale à la valeur de pH du couple de valeurs pH/conductivité mesurées additionnée de la valeur d'augmentation du pH résultant de la comparaison des données de pH et de conductivité de référence avec le couple de valeurs de pH/conductivité mesurées.

12. Installation (1) selon l'une des revendications 10 ou 11, **caractérisée en ce que** l'installation (1) comprend au moins un dispositif (7) d'émission d'un signal d'alerte sonore et/ou lumineux et **en ce que** l'unité (5) de pilotage est configurée pour commander l'émission d'un signal d'alerte sonore et/ou lumineux par le dispositif (7) d'émission d'un signal d'alerte sonore et/ou lumineux lorsque la valeur de pH de consigne est atteinte.

13. Installation selon l'une des revendications 10 à 12, **caractérisée en ce que** le système (2) de collecte de jus comprenant un réservoir (201) raccordable en entrée à la chambre (12) de pressurage, ledit réservoir (201) est équipé en sortie d'une première voie (202) de circulation et d'une seconde voie (203) de circulation sélectivement obturables, et l'unité (5) de pilotage est configurée pour commander l'obturation de l'une des voies de circulation et l'ouverture de l'autre voie de circulation lorsque la valeur de pH de consigne est atteinte.

14. Installation (1) selon l'une des revendications 10 à 13, **caractérisée en ce que** le système (2) de collecte de jus comprenant un réservoir (201) raccordable en entrée à la chambre (12) de pressurage, ladite entrée (2010) du réservoir (201) est équipée d'un organe (2011) d'obturation et l'unité (5) de pilotage est configurée pour commander la fermeture de l'organe (2011) d'obturation de ladite entrée (2010) lorsque la valeur de pH de consigne est atteinte.

15. Installation (1) selon l'une des revendications 10 à 14, **caractérisée en ce que** le pressurage comprenant une succession de paliers de pression, avec chaque palier correspondant à l'application d'une pression pendant une période de temps, ladite installation (1) comprend au moins un système (8) de détermination de l'avancement du pressurage et **en ce que** l'unité (5) de pilotage est configurée pour mesurer le pH et la conductivité des jus collectés en aval de la chambre (12) de pressurage pour enregistrer un couple de valeurs pH /conductivité en fonction de l'avancement dudit pressurage.

16. Installation (1) selon la revendication 15, **caractérisée en ce que** la chambre (12) de pressurage étant une chambre de volume variable apte à presser les raisins par variation de son volume, le système (8) de détermination de l'avancement du pressurage comprend au moins un organe (81) de mesure directe ou indirecte de l'état de la déformation de la chambre (12) de pressurage.

17. Installation (1) selon la revendication 16, **caractérisée en ce que** la chambre (12) de pressurage étant une chambre délimitée au moins partiellement par une membrane (14) déformable en fonction de la pression intérieure d'une chambre de commande (13) séparée de la chambre (12) de pressurage au moins par ladite membrane (14), le ou au moins l'un des organes (81) de mesure directe ou indirecte de l'état de la déformation de la chambre du système (8) de détermination de l'avancement du pressurage est un organe de mesure de la pression de préférence à l'intérieur de ladite chambre de commande (13).

18. Installation (1) selon l'une des revendications 10 à 17, **caractérisée en ce que** le système (2) de collecte des jus qui comprend un réservoir (201) dit principal raccordable en entrée à la chambre (12) de pressurage comprend au moins un réservoir (204) dit auxiliaire pour le stockage d'agents dits de collage à fonction d'agent de floculation ou de coagulation d'au moins une partie des composés desdits jus de raisin, et un système de transfert (205) du contenu du réservoir auxiliaire (204) vers le réservoir principal (201).

19. Installation (1) selon la revendication 18, **caractérisée en ce que** l'installation (1) comprend un dispositif de mesure de la quantité de jus collectés dans le réservoir (201) principal, tel qu'un débitmètre (82), et l'unité (5) de pilotage est configurée pour commander le système de transfert (205) en fonction au moins des données fournies par le dispositif de mesure.

20. Ensemble comprenant un pressoir (11) comprenant une chambre (12) de pressurage et une installation (1) de détermination , en aval de ladite chambre (12) de pressurage du pressoir, de la nature de jus de raisin produits au moins par pressurage de raisin dans ladite chambre (12) de pressurage en vue de distinguer les jus issus de la zone de la pulpe des grains de raisin des jus issus de la zone de la pellicule des grains de raisin, ladite installation (1) comprenant au moins, un système (2) de collecte de jus disposé en aval de la chambre (12) de pressurage et apte à être en communication fluidique avec la chambre (12) de pressurage pour collecter au moins les jus produits par pressurage du raisin dans la chambre (12) de pressurage, au moins un capteur (3) de mesure de pH disposé au niveau du système (2) de collecte, au moins une mémoire (4) de stockage de données et une unité (5) de pilotage configurée pour commander le stockage d'une valeur de pH de consigne dans la ou l'une des mémoires (4) et la mesure en temps réel et en continu du pH au niveau du système (2) de collecte au moins jusqu'à obtention de ladite valeur de pH de consigne mémorisée, **caractérisé en ce que** ladite installation (1) est conforme à l'une des revendications 10 à 19.

## Patentansprüche

1. Verfahren zur Bestimmung, stromab einer Presskammer (12) einer Presse (11), der Art von Mosten, die mindestens durch Pressen von Trauben in der Presskammer (12) erzeugt werden, um die aus dem Bereich des Fruchtfleisches der Traubenbeeren hervorgegangenen Moste von den aus dem Bereich der Schale der Traubenbeeren hervorgegangenen Moste zu unterscheiden, wobei das Verfahren mindestens einen Schritt des Speicherns eines Soll-pH-Werts und einen Schritt des kontinuierlichen und Echtzeit-Messens des pH-Werts der stromab der Presskammer (12) aufgefangenen Moste mindestens bis zum Erhalten des gespeicherten Soll-pH-Werts umfasst, **dadurch gekennzeichnet, dass** das Verfahren, vor dem Schritt des Speicherns des Soll-pH-Werts, einen Schritt des Messens des pH-Werts und der Leifähigkeit der stromab der Presskammer (12) aufgefangenen Moste, um ein Paar von pH-/Leitfähigkeitswerten zu registrieren, und einen Schritt des Bestimmens des zu speichernden Soll-pH-Werts in Abhängigkeit von dem gemessenen Paar pH-Wert/Leitfähigkeit umfasst.

2. Verfahren nach Anspruch 1, **dadurch gekennzeichnet, dass** es, vor dem Schritt des Speicherns des Soll-pH-Werts, einen Schritt des Speicherns von Referenz-pH- und -Leitfähigkeitsdaten umfasst und dass das Verfahren während des Schritts des Bestimmens des zu speichernden pH-Sollwerts in Abhängigkeit von dem gemessenen Paar pH-Wert/Leitfähigkeit eine Phase des Vergleichens der Referenz-pH- und -Leitfähigkeitsdaten mit dem Paar gemessener pH-/Leitfähigkeitswerte und eine Phase des Bestimmens einer Anhebung des pH-Werts in Abhängigkeit von dem Vergleich umfasst, wobei der zu speichernde Soll-pH-Wert gleich dem pH-Wert des Paars von gemessenen pH-/Leitfähigkeitswerten ist, zu dem der Wert der Anhebung des pH-Werts addiert wird, der aus dem Vergleich der Referenz-pH- und -Leitfähigkeitsdaten mit dem Paar gemessener pH-/Leitfähigkeitswerte resultiert.

3. Verfahren nach einem der Ansprüche 1 oder 2, **dadurch gekennzeichnet, dass** es einen Schritt des Sendens eines akustischen und/oder optischen Warnsignals umfasst, wenn der Soll-pH-Wert erreicht ist.

4. Verfahren nach einem der Ansprüche 1 bis 3, **dadurch gekennzeichnet, dass**, wenn die mindestens durch Pressen von Trauben in der Presskammer (12) erzeugten Moste stromab der Presskammer (12) entlang eines ersten (202) oder eines zweiten Zirkulationswegs (203) aufgefangen werden, das Verfahren einen Schritt des Änderns des Zirkulationswergs umfasst, wenn der Soll-pH-Wert erreicht ist.

5. Verfahren nach einem der Ansprüche 1 oder 4, **dadurch gekennzeichnet, dass** das Verfahren einen Schritt des Unterbrechens des Schritts des Auffangens der Moste umfasst, wenn der Soll-pH-Wert erreicht ist.

6. Verfahren nach einem der Ansprüche 1 bis 5, **dadurch gekennzeichnet, dass**, wenn die Presskammer (12) eine Kammer mit variablem Volumen ist, die geeignet ist die Trauben durch Änderung ihres Volumens zu pressen, und das Pressen eine Abfolge von Druckstufen umfasst, wobei jede Stufe der Ausübung eines Drucks entspricht, der aus einer Volumenverringerung der Presskammer während eines Zeitraums resultiert, das Verfahren mindestens einen Schritt des Bestimmens des Pressfortschritts umfasst und dass der Schritt des Messens des pH-Werts und der Leitfähgkeit der stromab der Presskammer aufgefangenen Moste zum Registrieren eines Paars von pH-/Leitfähigkeitswerten in Abhängigkeit von dem Pressfortschritt ausgeführt wird.

7. Verfahren nach Anspruch 6, **dadurch gekennzeichnet, dass** das Verfahren, während des Schritts des Bestimmens des Pressfortschritts mindestens eine Phase des direkten oder indirekten Messens des Zustands der Verformung der Presskammer (12) umfasst.

8. Verfahren nach einem der Ansprüche 6 oder 7, **dadurch gekennzeichnet, dass**, wenn die Presskammer (12) mit variablem Volumen eine Kammer ist, die mindestens teilweise durch eine Membran (14) begrenzt wird, die in Abhängigkeit von dem Innendruck einer Steuerkammer (13) verformbar ist, die von der Presskammer (12) mindestens durch die Membran (14) getrennt ist, das Verfahren, während des Schritts des Bestimmens des Pressfortschritts mindestens eine Phase des Messens des Drucks im Inneren der Steuerkammer (13) umfasst.

9. Verfahren nach einem der Ansprüche 1 bis 8, **dadurch gekennzeichnet, dass** das Verfahren mindestens einen Schritt des Zugebens zu den stromab der Presskammer (12) aufgefangenen Mosten mindestens eines Schönungsmittels mit Flockungs- oder Koagulationsmittelfunktion für mindestens einen Teil der Verbindungen der Traubenmoste umfasst.

10. Anlage (1) zur Bestimmung, stromab eine Presskammer (12) einer Presse (11), der Art von Traubenmosten, die mindestens durch Pressen von Trauben in der Presskammer (12) erzeugt werden, um die aus dem Bereich des Fruchtfleisches der Traubenbeeren hervorgegangenen Moste von den aus dem Bereich der Schade der Traubenbeeren hervorgegangenen Moste zu unterscheiden, die Anlage (1) mindestens umfassend ein System (2) zum Auffangen von Mosten umfasst, das stromab der Presskammer (12) angeordnet ist und geeignet ist, in Fluidverbindung mit der Presskammer (12) zu stehen, um die durch Pressen der Trauben in der Presskammer (12) erzeugten Moste aufzufangen, mindestens einen pH-Wert-Messsensor (3), der an dem System (2) zum Auffangen angeordnet ist, mindestens einen Speicher (4) zur Datenspeicherung und eine Ansteuerungseinheit (5), die dazu ausgestaltet ist, das Speichern eines Soll-pH-Werts in dem oder einem der Speicher (4) und das kontinuierliche Echtzeitmessen des pH-Werts an dem System (2) zum Auffangen mindestens bis zum Erhalten des gespeicherten Soll-pH-Werts zu steuern, **dadurch gekennzeichnet, dass** die Anlage (1), an dem System (2) zum Auffangen, einen Leitfähigkeitsmesssensor (6) umfasst, und dadurch, dass die Ansteuerungseinheit (5) dazu ausgestaltet ist, vor dem Steuern des Speicherns eines Soll-pH-Werts in dem oder einem der Speicher (4) das Messen, an dem System (2) zum Auffangen, des pH-Werts und der Leitfähigkeit zu steuern, um ein Paar von pH-/Leitfähigkeitswerten zu registrieren und um den zu speichernden Soll-pH-Wert in Abhängigkeit von dem Paar von gemessenen pH-/Leitfähigkeitswerten zu bestimmen.

11. Anlage (1) nach Anspruch 10, **dadurch gekennzeichnet, dass** der oder mindestens einer der Speicher ein Speicher zum Speichern von Referenz-pH- und -Leitfähigkeitsdaten ist, und dadurch, dass die Ansteuerungseinheit (5) dazu ausgestaltet ist, die Referenz-pH- und - Leitfähigkeitsdaten mit dem Paar gemessener pH-/Leitfähigkeitswerte zu vergleichen und eine Anhebung des pH-Werts in Abhängigkeit von dem Vergleich zu bestimmen, wobei der zu speichernde Soll-pH-Wert gleich dem pH-Wert des Paars von gemessenen pH-/Leitfähigkeitswerten ist, zu dem der Wert der Anhebung des pH-Werts addiert wird, der aus dem Vergleich der Referenz-pH- und - Leitfähigkeitsdaten mit dem Paar von gemessenen pH-/Leitfähigkeitswerten resultiert.

12. Anlage (1) nach einem der Ansprüche 10 oder 11, **dadurch gekennzeichnet, dass** die Anlage (1) mindestens eine Vorrichtung (7) zum Aussenden eines akustischen und/oder optischen Warnsignals umfasst und dass die Ansteuerungseinheit (5) dazu ausgestaltet ist, das Aussenden eines akustischen und/oder optischen Warnsignals durch die Vorrichtung (7) zum Aussenden eines akustischen und/oder optischen Warnsignals zu steuern, wenn der Soll-pH-Wert erreicht ist.

13. Anlage nach einem der Ansprüche 10 bis 12, **dadurch gekennzeichnet, dass** das System (2) zum Auffangen von Most einen Tank (201) umfasst, der eingangsseitig an die Presskammer (12) anschließbar ist, wobei der Tank (201) ausgangsseitig mit einem ersten Zirkulationsweg (202) und einem zweiten Zirkulationsweg (203 ausgestattet ist, die selektiv verschließbar sind, und wobei die Ansteuerungseinheit (5) dazu ausgestaltet ist, das Verschließen des einen der Zirkulationswege und das Öffnen des anderen Zirkulationswegs zu steuern, wenn der Soll-pH-Wert erreicht ist.

14. Anlage (1) nach einem der Ansprüche 10 bis 13, **dadurch gekennzeichnet, dass** das System (2) zum Auffangen von Most einen Tank (201) umfasst, der eingangsseitig an die Presskammer (12) anschließbar ist, wobei der Eingang (2010) des Tanks (201) mit einem Verschlussorgan (2011) ausgestattet ist und wobei die Ansteuerungseinheit (5) dazu ausgestaltet ist, das Schließen des Verschlussorgans (2011) des Eingangs (2010) zu steuern, wenn der Soll-pH-Wert erreicht ist.

15. Anlage (1) nach einem der Ansprüche 10 bis 14, **dadurch gekennzeichnet, dass** das Pressen eine Abfolge von Druckstufen umfasst, wobei jede Stufe der Ausübung eines Drucks während eines Zeitraums entspricht, die Anlage (1) mindestens ein System (8) zur Bestimmung des Pressfortschritts umfasst und die Ansteuerungseinheit (5) dazu ausgestaltet ist, den pH-Wert und die Leitfähigkeit der stromab der Presskammer (12) aufgefangenen Moste zu messen, um ein Paar von pH-/Leitfähigkeitswerten in Abhängigkeit von dem Pressfortschritt zu registrieren.

16. Anlage (1) nach Anspruch 15, **dadurch gekennzeichnet, dass**, wenn die Presskammer (12) eine Kammer mit variablem Volumen ist, die geeignet ist, die Trauben durch Änderung ihres Volumens zu pressen, dass System (8) zur Bestimmung des Pressfortschritts mindestens ein Organ (81) zum direkten oder indirekten Messen des Zustands der Verformung der Presskammer (12) umfasst.

17. Anlage (1) nach Anspruch 16 **dadurch gekennzeichnet, dass**, wenn die Presskammer (12) eine Kammer ist, die mindestens teilweise durch eine Membran (14) begrenzt wird, die in Abhängigkeit von dem Innendruck einer Steuerkammer (13) verformbar ist, die von der Presskammer (12) mindestens durch die Membran (14) getrennt ist, das oder mindestens eines der Organe (81) zum direkten oder indirekten Messen des Zustands der Verformung der Kammer des Systems (8) zur Bestimmung des Pressfortschritts ein organ zum Messen des Drucks, bevorzugt im Inneren der Steuerkammer (13), ist.

18. Anlage (1) nach einem der Ansprüche 10 bis 17, **dadurch gekennzeichnet, dass** das System (2) zum Auffangen der Moste, das einen Tank (201), Haupttank genannt, umfasst, der eingangsseitig an die Presskammer (12) anschließbar ist, mindestens einen Tank (204), Hilfstank genannt, zum Speichern von sogenannten Schönungsmitteln mit Flockungs- oder Koagulationsmittelfunktion für mindestens einen Teil der Verbindungen der Traubenmoste und ein Transfersystem (205) zum Transferieren des Inhalts des Hilfstanks (204) zu dem Haupttank (201) umfasst.

19. Anlage (1) nach Anspruch 18, **dadurch gekennzeichnet, dass** die Anlage (1) eine Vorrichtung zum Messen der Menge von in dem Haupttank (201) aufgefangenen Mosten wie einen Durchflussmesser (82) umfasst und die Ansteuerungseinheit (5) dazu ausgestaltet ist, das Transfersystem (205) in Abhängigkeit mindestens von den von der Messvorrichtung bereitgestellten Daten zu steuern.

20. Anordnung, umfassend eine Presse (11), die eine Presskammer (12) umfasst, und eine Anlage (1) zur Bestimmung, stromab der Presskammer (12) der Presse, der Art von Traubenmosten, die mindestens durch Pressen von Trauben in der Presskammer (12) erzeugt werden, um die aus dem Bereich des Fruchtfleisches der Traubenbeeren hervorgegangenen Moste von den aus dem Bereich der Schade der Traubenbeeren hervorgegangenen Moste zu unterscheiden, wobei die Anlage (1) mindestens ein System (2) zum Auffangen von Mosten umfasst, das stromab der Presskammer (12) angeordnet ist und geeignet ist, in Fluidverbindung mit der Presskammer (12) zu stehen, um mindestens die durch Pressen der Trauben in der Presskammer (12) erzeugten Moste aufzufangen, mindestens einen pH-Wert-Messsensor (3), der an dem System (2) zum Auffangen angeordnet ist, mindestens einen Speicher (4) zur Datenspeicherung und eine Ansteuerungseinheit (5), die dazu ausgestaltet ist, das Speichern eines Soll-pH-Werts in dem oder einem der Speicher (4) und das kontinuierliche Echtzeitmessen des pH-Werts an dem System (2) zum Auffangen mindestens bis zum Erhalten des gespeicherten Soll-pH-Werts zu steuern, datenelement die Anlage (1) eine Anlage nach einem der Ansprüche 10 bis 19 ist.

## Claims

1. Method for determining, downstream of a pressing chamber (12) of a press (11), the nature of juices produced at least by pressing grapes in said pressing chamber (12) with a view to distinguishing the juices originating from the zone of the pulp of the grape berries from the juices originating from the zone of the skin of the grape berries, said method comprising at least a step of storing a setpoint pH value and a step of real-time and continuous measurement of the pH of the juices collected downstream of the pressing chamber (12) at least until said stored setpoint pH value is obtained, **characterized in that** said method comprises, before the step of storing said setpoint pH value, a step of measuring the pH and the conductivity of the juices collected downstream of the pressing chamber (12) in order to record a pair of pH/conductivity values and a step of determining the setpoint pH value to be stored as a function of the measured pH/conductivity pair.

2. Method according to Claim 1, **characterized in that** it comprises, before the step of storing the setpoint pH value, a step of storing reference pH and conductivity data, and **in that** the method comprises, during the step of determining the setpoint pH value to be stored as a function of the measured pH/conductivity pair, a phase of comparing the reference pH and conductivity data with the pair of measured pH/conductivity values and a phase of determining an increase in the pH as a function of the comparison, the setpoint pH value to be stored being equal to the pH value of the pair of measured pH/conductivity values plus the pH increase value resulting from the comparison of the reference pH and conductivity data with the pair of measured pH/conductivity values.

3. Method according to either of Claims 1 and 2, **characterized in that** it comprises a step of emitting an audible and/or luminous alert signal when the setpoint pH value is reached.

4. Method according to one of Claims 1 to 3, **characterized in that**, the juices produced at least by pressing grapes in said pressing chamber (12) being collected downstream of the pressing chamber (12) by following a first (202) or a second circulation path (203), the method comprises a step of changing the circulation path when the pH setpoint value is reached.

5. Method according to one of Claims 1 to 4, **characterized in that** the method comprises a step of interrupting the step of collecting the juices when the setpoint pH value is reached.

6. Method according to one of Claims 1 to 5, **characterized in that**, said pressing chamber (12) being a chamber of variable volume that is able to press the grapes by varying its volume and the pressing comprising a succession of pressure stages, with each stage corresponding to the application of a pressure resulting from a reduction in volume of the pressing chamber for a period of time, said method comprises at least one step of determining the progress of the pressing and **in that** the step of measuring the pH and the conductivity of the juices collected downstream of the pressing chamber in order to record a pair of pH/conductivity values is carried out as a function of the progress of said pressing.

7. Method according to Claim 6, **characterized in that** the method comprises, during the step of determining the progress of the pressing, at least one phase of direct or indirect measurement of the state of the deformation of the pressing chamber (12).

8. Method according to either of Claims 6 and 7, **characterized in that**, the pressing chamber (12) of variable volume being a chamber delimited at least partially by a membrane (14) that is deformable as a function of the internal pressure of a control chamber (13) separated from the pressing chamber (12) at least by said membrane (14), the method comprises, during the step of determining the progress of the pressing, at least one phase of measuring the pressure inside said control chamber (13).

9. Method according to one of Claims 1 to 8, **characterized in that** the method comprises at least one step of adding to the juices collected downstream of the pressing chamber (12) at least one agent referred to as binding agent having the function of flocculating or coagulating agent for at least some of the compounds of said grape juices.

10. Installation (1) for determining, downstream of a pressing chamber (12) of a press (11), the nature of grape juices produced at least by grape pressing in said pressing chamber (12) with a view to distinguishing the juices originating from the zone of the pulp of the grape berries from the juices originating from the zone of the skin of the grape berries, said installation (1) comprising at least a juice collection system (2) disposed downstream of the pressing chamber (12) and able to be in fluidic communication with the pressing chamber (12) in order to collect the juices produced by pressing the grape in the pressing chamber (12), at least one pH measurement sensor (3) disposed at the collection system (2), at least one data storage memory (4) and a control unit (5) configured to control the storage of a setpoint pH value in the or one of the memories (4) and the real-time and continuous measurement of the pH at the collection system (2) at least until said stored setpoint pH value is obtained, **characterized in that** the installation (1) comprises, at the collection system (2), a sensor (6) for measuring the conductivity and **in that** the control unit (5) is configured to, prior to the control of the storage of a setpoint pH value in the or one of the memories (4), control the measurement, at the collection system (2), of the pH and of the conductivity in order to record a pair of pH/conductivity values and in order to determine the setpoint pH value to be stored as a function of the pair of measured pH/conductivity values.

11. Installation (1) according to Claim 10, **characterized in that** the or at least one of the memories is a memory for storing reference pH and conductivity data, and **in that** the control unit (5) is configured to compare the reference pH and conductivity data with the pair of measured pH/conductivity values and to determine an increase in the pH as a function of the comparison, the setpoint pH value to be stored being equal to the pH value of the pair of measured pH/conductivity values plus the pH increase value resulting from the comparison of the reference pH and conductivity data with the pair of measured pH/conductivity values.

12. Installation (1) according to either of Claims 10 and 11, **characterized in that** the installation (1) comprises at least one device (7) for emitting an audible and/or luminous alert signal and **in that** the control unit (5) is configured to control the emission of an audible and/or luminous alert signal by the device (7) for emitting an audible and/or luminous alert signal when the setpoint pH value is reached.

13. Installation according to one of Claims 10 to 12, **characterized in that**, the juice collection system (2) comprising a tank (201) that can be connected at the inlet to the pressing chamber (12), said tank (201) is equipped at the outlet with a first circulation path (202) and a second circulation path (203) that can be selectively shut off, and the control unit (5) is configured to control the shutting-off of one of the circulation paths and the opening of the other circulation path when the setpoint pH value is reached.

14. Installation (1) according to one of Claims 10 to 13, **characterized in that**, the juice collection system (2) comprising a tank (201) that can be connected at the inlet to the pressing chamber (12), said inlet (2010) of the tank (201) is equipped with a shut-off member (2011) and the control unit (5) is configured to control the closing of the shut-off member (2011) of said inlet (2010) when the setpoint pH value is reached.

15. Installation (1) according to one of Claims 10 to 14, **characterized in that**, the pressing comprising a succession of pressure stages, with each stage corresponding to the application of a pressure for a period of time, said installation (1) comprises at least one system (8) for determining the progress of the pressing and **in that** the control unit (5) is configured to measure the pH and the conductivity of the juices collected downstream of the pressing chamber (12) in order to record a pair of pH/conductivity values as a function of the progress of said pressing.

16. Installation (1) according to Claim 15, **characterized in that**, the pressing chamber (12) being a chamber of variable volume that is able to press the grapes by varying its volume, the system (8) for determining the progress of the pressing comprises at least one member (81) for directly or indirectly measuring the state of the deformation of the pressing chamber (12).

17. Installation (1) according to Claim 16, **characterized in that**, the pressing chamber (12) being a chamber delimited at least partially by a membrane (14) that is deformable as a function of the internal pressure of a control chamber (13) separated from the pressing chamber (12) at least by said membrane (14), the or at least one of the members (81) for directly or indirectly measuring the state of the deformation of the chamber of the system (8) for determining the progress of the pressing is a member for measuring the pressure preferably inside said control chamber (13).

18. Installation (1) according to one of Claims 10 to 17, **characterized in that** the system (2) for collecting the juices that comprises a tank (201) referred to as main tank that can be connected at the inlet to the pressing chamber (12) comprises at least one tank (204) referred to as auxiliary tank for storing agents referred to as binding agents having the function of flocculating or coagulating agents for at least some of the compounds of said grape juices, and a system (205) for transferring the contents of the auxiliary tank (204) to the main tank (201).

19. Installation (1) according to Claim 18, **characterized in that** the installation (1) comprises a device for measuring the quantity of juices collected in the main tank (201), such as a flow meter (82), and the control unit (5) is configured to control the transfer system (205) as a function at least of the data supplied by the measurement device.

20. Assembly comprising a press (11) comprising a pressing chamber (12) and an installation (1) for determining, downstream of said pressing chamber (12) of the press, the nature of grape juices produced at least by grape pressing in said pressing chamber (12) with a view to distinguishing the juices originating from the zone of the pulp of the grape berries from the juices originating from the zone of the skin of the grape berries, said installation (1) comprising at least a juice collection system (2) disposed downstream of the pressing chamber (12) and able to be in fluidic communication with the pressing chamber (12) in order to collect at least the juices produced by pressing the grape in the pressing chamber (12), at least one pH measurement sensor (3) disposed at the collection system (2), at least one data storage memory (4) and a control unit (5) configured to control the storage of a setpoint pH value in the or one of the memories (4) and the real-time and continuous measurement of the pH at the collection system (2) at least until said stored setpoint pH value is obtained, **characterized in that** said installation (1) is in accordance with one of Claims 10 to 19.
